(19) **Europäisches Patentamt / European Patent Office / Office européen des brevets**

(11) **EP 3 727 300 B2**

(12) **NEW EUROPEAN PATENT SPECIFICATION**
After opposition procedure

(45) Date of publication and mention
of the opposition decision:
**29.07.2026 Bulletin 2026/31**

(45) Mention of the grant of the patent:
**26.10.2022 Bulletin 2022/43**

(21) Application number: **18825643.2**

(22) Date of filing: **17.12.2018**

(51) International Patent Classification (IPC):
*A61K 8/34* (2006.01)   *A61K 8/41* (2006.01)
*A61Q 11/00* (2006.01)   *A61Q 17/00* (2006.01)
*A61K 8/9789* (2017.01)

(52) Cooperative Patent Classification (CPC):
A61Q 11/00; A61K 8/347; A61K 8/416;
A61K 8/9789; A61K 31/05; A61Q 17/005;
A61K 2800/70; Y02A 50/30

(86) International application number:
**PCT/EP2018/085103**

(87) International publication number:
**WO 2019/121440 (27.06.2019 Gazette 2019/26)**

(54) **AN ANTIMICROBIAL COMPOSITION**

ANTIMIKROBIELLE ZUSAMMENSETZUNG

COMPOSITION ANTIMICROBIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2017 EP 17210127**

(43) Date of publication of application:
**28.10.2020 Bulletin 2020/44**

(73) Proprietors:
• **Unilever Global IP Limited**
**Wirral, Merseyside CH62 4ZD (GB)**
Designated Contracting States:
**CY DE GB IE IT MT**
• **Unilever IP Holdings B.V.**
**6708 WH Wageningen (NL)**
Designated Contracting States:
**AL AT BE BG CH CZ DK EE ES FI FR GR HR HU IS LI LT LU LV MC MK NL NO PL PT RO RS SE SI SK SM TR**

(72) Inventors:
• **APPAVOO, Shanthi**
**Bangalore 560 066, Karnataka (IN)**
• **DASGUPTA, Anindya**
**Bangalore 560 066, Karnataka (IN)**
• **MEDEPALLI, Srilaxmi, Venkata**
**Bangalore 560 066, Karnataka (IN)**
• **NAIR, Rohini, Sukumaran**
**Bangalore 560 066, Karnataka (IN)**
• **SAJI, Maya, Treesa**
**Bangalore 560 066, Karnataka (IN)**
• **WASKAR, Morris**
**Bangalore 560 066, Karnataka (IN)**

(74) Representative: **Unilever Patent Group**
**Bronland 14**
**6708 WH Wageningen (NL)**

(56) References cited:
WO-A1-01/82922     WO-A1-2017/194256
US-A1- 2014 328 772     US-A1- 2015 157 542

EP 3 727 300 B2

**Description**

**Field of the invention**

The present invention relates to an antimicrobial composition.

**Background of the invention**

People try to take good care of external surfaces of their bodies as well as those of their pets to maintain good overall health. These surfaces include their skin, oral cavity and scalp. Specific skin related issues that people are concerned about include healthy and infection-free skin, good skin tone and adequate moisturization. Oral cavity is another external surface that people actively take care to maintain. People generally prefer thick long hair with minimum hair fall and an infection-free scalp.

Many if not all problems related to skin, oral cavity or scalp, are linked to microorganisms like e.g. bacteria and some fungi. Some bacteria like *Escherichia coli (E. coli)* and *Staphylococcus aureus* (*S. aureus*) are commonly found on the human skin. These bacteria *per se* are not pathogenic whilst being commonly present on the skin. However, when they enter the human body through acts like ingestion, these bacteria tend to become pathogenic. Much of problems of the oral cavity including gums and teeth, like e.g. cavities, tartar, gingivitis, caries, bad breath also known as halitosis and plaque are associated with presence of bacteria in the oral cavity. In addition, dandruff, a commonly occurring scalp problem, also is an implication of scalp infected by a fungal microorganism.

Therefore, people often tend to take care of their external surfaces by keeping them free of microorganisms. One way to tackle infections is to treat them with antimicrobial actives after the infection has set in. Whereas, another approach is to leave on these surfaces a minimal amount of an antimicrobial active so that any invading microorganisms are killed or inactivated thereby minimizing spread of an infection.

A few references are found in prior art that relate to certain actives and antimicrobial benefit obtained therefrom.

WO11038797 (Beiersdorf AG, 2009) discloses use of magnolol or honokiol as antibacterial, antimycotic, antiparasitic or antiviral active ingredients, wherein only one of the two active ingredients are used in each case.

US2009156551 (DSM, 2009) discloses compositions comprising magnolol and honokiol wherein the mol ratio of magnolol to honokiol is less than 0.6 as well as to the use of these compositions as medicament, in particular as a medicament for the treatment, co-treatment or prevention of inflammatory disorders.

WO0182922 (Procter and Gamble, 2001) discloses oral compositions comprising an effective amount of a polyphenol herbal extract selected from the group consisting of magnolol, honokiol, tetrahydromagnolol, tetrahydrohonokiol, and mixtures thereof; an effective amount of a buffering agent; from about 40 percent to about 99 percent of one or more aqueous carriers; wherein the oral composition has a total water content of from about 5 percent to about 70 percent.

US 2014/328772 (Colgate - Palmolive Co.) discloses a composition comprising a solubilized magnolol analog comprising at least one magnolol analog chosen from propyl magnolol, isopropyl magnolol, butyl magnolol and isobutyl magnolol along with a sorbitan-ester. US 2014/328772 relates to solving a problem of solubilizing said magnolol analogs using sorbitan esters e.g. polysorbate 20, polysorbate 40, polysorbate 60, polysorbate 80 and polysorbate 85, that selectively solubilize said magnolol analogs.

WO 2017/194256 A1 (Unilever) discloses a topical composition for antimicrobial benefit and a method of disinfecting a surface. According to the invention there is provided a topical composition comprising: a) 0.1 to 10 % by weight of Niacinamide or its derivatives; b) 0.01% to 5% by weight of a first quaternary ammonium salt comprising Didecyl Dimethyl Ammonium Chloride; c) 0.01 to 5 % by weight of a second quaternary ammonium salt; and d) a cosmetically acceptable base.

Despite efforts thus far, people are always on a look out for new technologies like e.g. actives or combination of actives that will deliver antimicrobial benefit. Further, it is preferred if minimal amounts of known antimicrobial actives are used as people nowadays prefer minimum exposure to chemical ingredients. Therefore, antimicrobial compositions and actives that deliver an antimicrobial benefit, remains a topic of interest.

Need therefore exists to provide an antimicrobial composition comprising one or more actives that delivers an antimicrobial benefit. Need also exists to provide antimicrobial composition that comprises minimal amount of known antimicrobial actives.

It is an object of the present invention to provide an antimicrobial composition.

It is another object of the present invention to provide an antimicrobial composition with minimal amount of known antimicrobial actives.

The present inventors have surprisingly found that a combination of at least one compound selected from biphenol and at least one antimicrobial quaternary ammonium compound, provides synergistic antimicrobial benefit. It has also been found that such antimicrobial benefit is obtained using minimal amounts of known antimicrobial actives. It has also been found that such antimicrobial benefit is obtained in relatively short contact times against gram positive and gram negative microorganisms.

**Summary of the invention**

In a first aspect, the present invention relates to an antimicrobial composition according to claim 1.

In a second aspect, the present invention relates to a non-therapeutic method of cleaning or disinfecting a surface comprising the step of applying a composition of the first aspect on to a surface.

In a third aspect, the present invention relates to non-therapeutic use of a composition of the first aspect for antimicrobial benefit.

In a fourth aspect, the present invention relates to a composition of the first aspect for use in a method to improve hand hygiene.

Detailed description of the invention

Any feature of one aspect of the present invention may be utilized in any other aspect of the invention. The word "comprising" is intended to mean "including" but not necessarily "consisting of' or "composed of." In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

In a first aspect, the present invention relates to an antimicrobial composition according to claim 1.

Antimicrobial composition as mentioned herein above preferably means any composition, which is capable of killing or at least cause substantial reduction of the common disease causing microbes. The common disease causing gram-positive organisms includes *Staphylococcus, Streptococcus* and *Enterococcus* spp. Some of common disease causing gram-negative organisms includes *Escherichia coli, Salmonella, Klebsiella* and *Shigella. Escherichia coli* and *Salmonella* can cause severe gastrointestinal illnesses.

Biphenol

The composition as per the present invention (the composition) comprises at least one biphenol wherein the biphenol is a diallylbiphenol. This means the biphenol comprises diallyl substitution.

Examples of biphenol that are suitable for use in the present invention include 2,4'-biphenol (IUPAC name: 2,4'-Dihydroxybiphenyl), 2,2'-biphenol (2,2'-Dihydroxybiphenyl), 3,3'-biphenol (3,3'-Dihydroxybiphenyl), 4,4'-biphenol (4,4'-Dihydroxybiphenyl), and mixtures thereof.

The diallyl substitution may be present at any of the positions on biphenol structure. The preferred diallylbiphenol is a (5-3'-diallyl)biphenol or a (5-5'-diallyl)biphenol. An example of (5-3'-diallyl)biphenol include 5-3'-diallyl-2,4'-dihydroxybiphenyl (commonly known as honokiol). An example of (5-5'-diallyl)biphenol include 5-5'-diallyl-2,2'-dihydroxybiphenyl (commonly known as magnolol). The most preferred diallylbiphenol is a (5-3'-diallyl)biphenol.

Preferably, the composition comprises from 0.001 to 10%, preferably from 0.005 to 8%, more preferably from 0.01 to 6%, even more preferably from 0.05 to 5%, further more preferably from 0.1 wt to 4%, still more preferably from 0.5 to 3% and yet more preferably from 1 to 1.5% by weight of a biphenol.

An antimicrobial quaternary ammonium compound:

The composition comprises at least one antimicrobial quaternary ammonium compound, wherein the quaternary ammonium compound is selected from benzethonium chloride (BEC), benzalkonium chloride (BKC), polydiallyldimethylammonium chloride (poly-DADMAC), didecyl dimethyl ammonium chloride (DDAC), cetylpyridinium chloride, cetyltrimethylammonium chloride (CTAC), and cetyltrimethylammonium bromide (CTAB).

Preferred antimicrobial compounds are any one of BEC, BKC, poly-DADMAC, DDAC, CTAC, CTAB and mixtures thereof. More preferred antimicrobial compounds are BEC, BKC, poly-DADMAC and mixtures thereof.

The composition comprises from 0.0001 to 5%, more preferably from 0.0005 to 5%, even more preferably from 0.001 to 5%, furthermore preferably from 0.005 to 5%, still more preferably from 0.01 to 4%, yet more preferably from 0.05 to 3%, even furthermore preferably from 0.1 to 2% and even furthermore preferably from 1 to 2% by weight of antimicrobial quaternary ammonium compound.

Preferably, the composition does not comprise poly(acrylamide-co-diallyldimethylammonium chloride), i.e. polyquaternium-7, as the antimicrobial quaternary ammonium compound.

Preferably, the composition does not comprise cocoamidopeopyl betain (CAPB) as the antimicrobial quaternary ammonium compound.

Preferably, the composition further comprises surfactant that can be selected from nonionic, anionic, cationic, amphoteric surfactant and mixtures thereof. More preferably, surfactant selected is nonionic surfactant.

Preferred examples of nonionic surfactants are those with a $C_9$-$C_{20}$ fatty alcohol or acid hydrophobe condensed with from 2 to 100 moles of ethylene oxide or propylene oxide per mole of hydrophobe; $C_2$-$C_{10}$ alkyl phenols condensed with from 2 to 20 moles of alkylene oxide; mono- and di- fatty acid esters of ethylene glycol; fatty acid monoglyceride; sorbitan, mono- and di- $C_8$-$C_{20}$ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants. Particulary, preferred nonionic surfactant is C9-C11 fatty alcohol having upto 6 moles of ethylene oxide. Such nonionic surfactant is available under NEODOL® 91-5 by Shell.

When incorporated in the compositions, nonionic surfactants may be present in an amount from 0.1 to 80%, preferably from 1 to 50%, more preferably from 3 to 30%, even more preferably from 5 to 20%, further more preferably from 10 to 15% by weight of the composition.

Preferably, the composition further comprises a cosmetically acceptable base. The cosmetically acceptable base is preferably in the form of a cream, lotion, gel or emulsion.

The compositions may be prepared using different cosmetically acceptable emulsifying or non-emulsifying base. A highly suitable base is a cream. Vanishing creams are especially preferred. Vanishing cream bases generally comprise a mixture of fatty acid and soap. Vanishing cream base gives a highly appreciated matty feel to the skin. C12 to C20 fatty acids are especially preferred in vanishing cream bases, further more preferred being C14 to C18 fatty acids. Most preferably, the fatty acids are stearic acid or palmitic acid or mixtures thereof. The fatty acid is often hystric acid which is substantially (generally from 90 to 95% by weight) a mixture of 45% stearic and 55% palmitic acid. Thus, inclusion of hystric acid and its soap to prepare compositions of the invention is within the scope of the present invention. The fatty acid in the composition is preferably present in an amount from 5 to 20%, more preferably from 6 to 19% and even more preferably from 7 to 17% by weight of the composition. Soaps in the vanishing cream base include alkali metal salt of fatty acids, like sodium or potassium salts, most preferred being potassium stearate. The soap in the vanishing cream base is generally present in an amount in the range from 0.1 to 10%, more preferably from 0.1 to 3% by weight of the composition. Generally, the vanishing cream base topical compositions are prepared by taking a desired amount of total fatty matter and mixing with potassium hydroxide in desired amounts. The soap is usually formed in-situ during the mixing.

An especially suitable cosmetically acceptable base is one which comprises a water-in-oil emulsion comprising silicone oils as the continuous phase. The water-in-oil emulsions preferably comprise a cross-linked silicone elastomer blend.

Inclusion of silicone elastomer blend in a water-in-oil emulsion may be used as the cosmetically acceptable base for preparing the compositions of the present invention. While silicone fluids may be used, silicone elastomers which are cross-linked, are especially preferred. In contrast to silicone fluid polymers, the physical properties of elastomers are typically dependent on the number of cross-linkages, rather than molecular weight. The ability of silicone elastomers to swell makes them ideal thickeners for oil phases. The elastomers have a very smooth and soft feel when applied to skin or hair. They can also be used as delivery agents for fragrances, vitamins and other additives in cosmetic compositions. Suitable silicone elastomer blends or gels which are commercially available and suitable for inclusion in the composition of the invention and found to provide the enhanced stability are: Dow Corning® EL-8051 IN Silicone Organic Elastomer Blend [INCI Name: Isodecyl Neopentanoate (and) Dimethicone/Bis Isobutyl PPG-20 Crosspolymer], EL-8050 [INCI Name: Isododecane (and) Dimethicone/Bis-Isobutyl PPG 20 Crosspolymer], DC9040, DC9041, DC9045 (Dimethicone crosspolymer), DC9506, DC9509 (Dimethicone vinyl dimethicone crosspolymer) and Shin-Etsu KSG-15, KSG-16, KSG-17 (Dimethicone vinyl dimethicone crosspolymer). It is further preferred that the composition comprises from 5 to 50% by weight silicone elastomer.

Preferably, the cosmetically acceptable base is present preferably from 10 to 99.9%, more preferably from 50 to 99%, even more preferably from 60 to 85% and further more preferably from 65 to 80% by weight of the composition.

Preferably, the composition further comprises skin lightening agents. Examples of skin lightening agents that may be used in the composition include, 12-hydroxystearic acid, aloe extract, ammonium lactate, arbutin, azelaic acid, kojic acid, butyl hydroxy anisole, butyl hydroxy toluene, citrate esters, 3 diphenyl propane derivatives, 2, 5 dihydroxybenzoic acid and its derivatives, ellagic acid, fennel extract, gluco pyranosyl-1-ascorbate, gluconic acid, glycolic acid, green tea extract, hydroquinone, 4 hydroxyanisole and its derivatives, 4-hydroxy benzoic acid derivatives, hydroxycaprylic acid, lemon extract, linoleic acid, magnesium ascorbyl phosphate, mulberry root extract, 2,4 resorcinol derivatives, 3,5 resorcinol derivatives, salicylic acid, vitamins like vitamin B6, vitamin B12, vitamin C, vitamin A, a dicarboxylic acid, resorcinol derivatives, hydroxycarboxylic acid like lactic acid and their salts e.g. sodium lactate and mixtures thereof.

Preferably, the composition further comprises one or more sunscreens. Any sunscreen that can be suitably used with the base may be added. Both, UVA and UVB sunscreens may preferably be added.

The composition of the invention may preferably comprise a UV- A sunscreen which is a dibenzoylmethane or its derivatives. Preferred dibenzoylmethane derivatives are selected from 4-tert-butyl-4'-methoxydibenzoylmethane, 2-methyldibenzoylmethane, 4-methyl-dibenzoylmethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxydibenzoyl methane, 2,4-dimethyl-4'- methoxy dibenzoylmethane or 2,6-dimethyl-4-tert-butyl-4'-methoxy-dibenzoylmethane. The most preferred dibenzoylmethane derivative is 4-tert-butyl-4'-methoxydibenzoylmethane. The composition of the invention preferably comprises from 0.1 to 10%, more preferably from 0.2 to 5%, further more preferably from 0.4 to 3%, by weight dibenzoylmethane or a derivative thereof based on total weight of the composition and including all ranges subsumed therein.

The composition may also preferably comprise a UV-B organic sunscreen selected from the class of cinnamic acid, salicylic acid, diphenyl acrylic acid and derivatives thereof. Illustrative non-limiting example of UV-B sunscreens which are commercially available and useful for inclusion in the composition of the invention are Octisalate™, Homosalate™, NeoHelipan™, Octocrylene™, Oxybenzone™ or Parsol MCX™. The UV-B sunscreen is most preferably 2-ethylhexyl-4-methoxy cinnamate which is commercially available as Parsol MCX™. The UV-B organic sunscreen is preferably included in the composition from .1 to 10%, more preferably from 0.1 to 7 % by weight of the composition. It has been observed that presence of an organic UV-B sunscreen like 2-ethylhexyl-4-methoxy cinnamate causes further rapid degradation of the UV-A dibenzoylmethane sunscreen in the presence of UV radiation. The presence of the rosmarinic acid ester compound is found to be very efficacious in stabilizing the composition even when UV-B sunscreens are present.

Useful inorganic sun-blocks are also preferably used in the present invention. These include, for example, zinc oxide, iron oxide, silica, such as fumed silica, and titanium dioxide.

The composition may further comprise preservatives to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts. Particularly preferred preservatives are phenoxyethanol, methyl paraben, propyl paraben, imidazolidinyl urea, sodium dehydroacetate, benzyl alcohol, alkane diols most preferably 1,2-octane diol and phenoxyethanol. The preservatives should be selected having regard for the use of the composition and possible incompatibility between the preservatives and other ingredients. Preservatives are preferably employed in amounts from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide, scrub and exfoliating particles such as polyethylene and silica or alumina; cooling agents such as menthol; skin calming agents such as aloe vera; and colorants.

In addition, the compositions may further comprise from 0 to 10% by weight of opacifiers and pearlizers such as ethylene glycol distearate, titanium dioxide or Lytron® 621 (Styrene/Acrylate copolymer); all of which are useful in enhancing the appearance or properties of the product.

Diluents other than water that may be used in the composition includes liquid or solid emollients, solvents, humectants, thickeners and powders. Examples of each of these types of vehicle, which can be used singly or as mixtures of one or more vehicles, are as follows:

Preferably, the composition comprises emollients. Exampels of emollients that may be present include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate.

Preferably, the composition comprises solvents such as ethyl alcohol, isopropanol, acetone, ethylene glycol monoethyl ether, diethylene glycol monobutyl ether, diethylene glycol monoethyl ether and mixtures thereof.

Advantageously, the composition may preferably comprise ingredients like bactericides, vitamins, anti-acne actives, anti-wrinkle, anti-skin atrophy and skin repair actives, skin barrier repair actives, non-steroidal cosmetic soothing actives, artificial tanning agents and accelerators, sebum stimulators, sebum inhibitors, anti-oxidants, protease inhibitors, skin tightening agents, anti-itch ingredients, hair growth inhibitors, 5-alpha reductase inhibitors, desquamating enzyme enhancers, anti-glycation agents and mixtures thereof.

The composition may preferably comprise powders like e.g. chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and mixtures thereof.

The compositions of the present invention can comprise a wide range of other optional components. The CTFA Personal care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, pH adjusters, natural extracts, skin sensates, skin soothing agents, and skin healing agents.

The composition may also comprise one or more of the following ingredients e.g. chloroxylenol, zinc pyrithione (ZPT), creatine and creatinine.

The composition is preferably in the form of a wash-off or a leave-on composition, preferably a leave-on composition.

Wash-off composition preferably means composition which is intended/required to be removed from the body by washing with solvent preferably water after the application of the composition like e.g. hand wash composition and face wash composition.

Leave-on composition preferably means composition which is not required to be removed from the human body after the application of the composition like e.g. skin cream, body lotion, hand sanitizer and deodorants.

When the composition is in the form of a leave-on composition, the composition may be in the form of a deodorant, a hand sanitizer, a lotion, a cream and a body spray.

The composition of the invention may preferably comprise a conventional deodorant base as the cosmetically acceptable carrier. By a deodorant is meant a product in the stick, roll-on, or propellant medium which is used for personal deodorant benefit e.g. application in the under-arm area which may or may not contain anti-perspirant actives.

Deodorant compositions can generally be in the form of firm solids, soft solids, gels, creams, and liquids and are dispensed using applicators appropriate to the physical characteristics of the composition. Deodorant compositions which are delivered through roll-ons generally comprise a liquid carrier. Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95% and in many instances from 40 to 80% by weight of the composition. Hydrophobic liquid carriers commonly can comprise one or more materials selected within the chemical classes of siloxanes, hydrocarbons, branched aliphatic alcohols, esters and ethers that have a melting point not higher than 25°C and a boiling point of at least 100°C. Hydrophilic carrier liquids that can be employed in compositions herein commonly comprise water and/or a mono or polyhydric alcohol or water-miscible homologue. Other than this suitable other vehicle and component used for deodorant composition can be added.

In a second aspect, the present invention relates to a non-therapeutic method of cleaning or disinfecting a surface comprising the steps of applying the composition described in the first aspect on to a surface in case of a leave-on composition. This method optionally comprises an additional step of at least partially removing the composition from the surface if it is in the form of a wash-off composition. Preferably, the step of at least partially removing the composition is carried out in less than 5 minutes after the step of applying the composition on to the substrate.

In a third aspect, the present invention relates to non-therapeutic use of the composition as described in the first aspect for antimicrobial benefit. An antimicrobial benefit preferably means after application of the composition, the residual microbes on the surface is significantly less. The composition of the present invention provides improved antimicrobial benefits.

In a fourth aspect, the present invention relates to the composition for use in a method to improve hand hygiene.

The present invention now will be demonstrated by way of following non-limiting examples.

**Examples**

Materials:

- Honokiol (98% pure from World Way Biotech, China),
- Magnolol (98% pure from World Way Biotech, China),
- 3,3'- Biphenol (Sigma; cat. No. S846708),
- Benzethonium chloride (BEC; Sigma, cat. No. B8879),
- Benzalkonium chloride (BKC; 50% solution; Sigma, cat. No. 63249),
- Polydiallyldimethylammonium chloride (Poly-DADMAC; available under Merquat™ 100 (40% solution) from Lubrizol),
- Trypticase soy agar (TSA; Difco cat. No. 236950); and
- Dey-Engley neutralizing broth (D/E; Difco cat. No. 281910),
- *Staphylococcus aureus* (*S. aureus* ATCC 6538),
- *Escherichia coli* (*E. coli* ATCC 10536); and
- *Staphylococcus hominis* (*S. hominis* ATCC 27844)

In-vitro experiments that demonstrate antimicrobial benefit upon treatment with a composition as per the present invention.

*Germ removal test:*

The experiment was done using the following protocol:

Compositions outlined in Tables 1 to 3 provided below were tested for germ removal test as per European suspension tests, EN1276. Briefly, dilutions of the desired test compositions were prepared in hard water in a 100 mL sample container. A test bacterial saline suspension containing $10^8$ cells / mL was prepared. 1 mL of the above test bacterial suspension was added to 1 mL of sterilized 3% by weight BSA solution (simulating high soil conditions) or sterilized 0.3% by weight BSA (simulating low soil conditions) and allowed to stand for 2 minutes. At the end of 2 minutes, the mixture was transferred into the sample container containing 8 mL solution of desired test compositions. At the end of desired contact time, like e.g. 5 minutes and 15 minutes, the mixture was vortexed and 1 mL of it was transferred to 9 mL of neutralizing broth (Dey-Engley (D/E) for gram negative organisms or Butterfield's Phosphate Buffer (BPB) medium for gram positive organisms) containing tube. Serial dilutions of this were carried out in D/E or BPB neutralizer for further enumeration. Plating was carried out using TSA medium. Results were recorded after 24 to 48 hours of incubation at 37°C.
The log reduction was calculated as:

$$\text{Log}_{10} \text{ Reduction (LR)} = \text{Log}_{10} \text{ of control - Log}_{10} \text{ of test sample.}$$

Log reduction greater than 5 means 99.99% reduction in the number of cells and log reduction less than 0.5 means no reduction, i.e. no antimicrobial benefit. Log reduction greater than 5 also denotes complete kill.

Table 1:

| Example | Honokiol | 3, 3' - Biphenol | BKC | BEC | Meraquat-100 | Average log reduction in *S. aureus* | SD |
|---|---|---|---|---|---|---|---|
| A | 0.02 | - | - | - | - | 0.31 | 0.06 |
|  |  |  |  |  |  |  |  |
| B | - | 0.02 | - | - | - | 0.30 | 0.27 |
| C | - | - | 0.005 | - | - | 0.58 | 0.28 |
| D | - | - | - | 0.005 | - | 1.07 | 0.28 |
| E | - | - | - | - | 0.002 | 1.23 | 0.17 |
| 1 | 0.02 | - | 0.005 | - | - | >5 | 0.42 |
| 2 | 0.02 | - | - | 0.005 | - | 4.59 | 0.67 |
| 3 | 0.02 | - | - | - | 0.002 | 4.24 | 0.08 |
| 4 | - | 0.02 | 0.005 | - | - | >5 | 0.34 |

From the above data it is evident that the Examples (1 to 4) that are within the scope of present invention provides much higher log reduction value. In some case (Example 1 and 4) 99.99% reduction is achieved. On the other hand the examples (A to E) that are outside the scope of the present invention does not provide much benefit at low concentration of known antimicrobial actives like BKC, BEC etc.

Long lasting hygiene benefit:

Further, the composition as per the present invention were found to provide long lasting hygiene (LLH) benefit. The LLH benefit was estimated using the following protocol:

Preparation of bacterial culture:

*E. coli* ATCC 10536 was sub-cultured on sterile TSA and grown overnight at 37°C. The optical density of cells was adjusted at 620 nm to get $1 \times 10^8$ cells / mL in a saline suspension.

Long lasting hygiene benefit:

Further, the composition as per the present invention were found to provide long lasting hygiene (LLH) benefit. The LLH benefit was estimated using the following protocol developed in-house:

Day 1: Treatment of a surface:

10 µL of 1 x $10^8$ cells/mL of *E. coli* (ATCC 10536) was spread over the whole surface of a pre-cleaned tile using a spreader and the tile was left to dry. 1 mL of treatments as shown in table 4 below were added to the tile and were spread. After a contact time of 10 minutes, excess was removed by tilting the tile and using a spatula to sweep off the excess. The tile was left to dry and stored overnight under professional care wipes (Wipertec, England).

Day 2: Wet and Dry cycle:

Wet cycle: Sterile distilled water was sprayed onto a wipe and the wipe was moved over the tile with light hand pressure. The tile was left to dry.
A dry cycle was carried out as above but the wipe was not wetted.
The tile was then rinsed by 5 mL of sterile water twice over the whole surface. The tile was left to dry. It was covered with professional care wipe and left overnight.

Day 3: Inoculation of the tile:

10 µL of the bacterial suspension prepared as described above was added to the tile and spread over the tile surface. It was left to dry. One dry/wet cycle was carried out as described above.
Final inoculation of tile: 10 µL of the bacterial suspension was applied to the tile as described before. After 10 minutes, 500 µL double strength neutralizer (D/E broth) was added and spread over entire surface. After 5 minutes the neutralizer was swept to the center of the tile and collected into a microtitre plate.
The sample was serially diluted to an appropriate level, using D/E neutralizing solution. 100 µL from each dilution was spread onto TSA agar plates. The plates were incubated at 37°C and the colonies counted after 24 to 48 h. The data on average residual colony forming units (CFU) log after treatments indicated in table 3 is provided below. As this is residual log, lower the log value, higher is the benefit.

Table 3

| Example | Water | Honokiol | BKC | C9-C11 fatty alcohol | Average Residual CFU log | SD |
|---------|-------|----------|-----|---------------------|--------------------------|-----|
| F | 100 | - | - | - | 6.49 | 0.22 |
| G | 99.25 | 0.25 | - | 0.5 | 6.66 | 0.08 |
| H | 98.5 | - | 1 | 0.5 | 5.11 | 0.74 |
| 5 | 98.25 | 0.25 | 1 | 0.5 | <u>2.83</u> | 0.60 |

The data in table 3 shows that almost 4 log reduction in CFU was obtained using the compositing as per the present invention (Example 5). The residual log in case of Example 5 is significantly less than the examples that are outside the scope of the present invention.

Antimicrobial benefit from honokiol and/or magnolol as estimated based on $H_2S$ reduction protocol

Antimicrobial benefit from honokiol and/or magnolol was estimated using a method described below and is based on method described in the literature (Clarke, P. H. 1953, J. gen. Microbiol. 8, 397-407). Some bacteria have the enzymatic capability to degrade amino acids like e.g. cysteine and cystine, that contain sulfhydryl group (-SH) thereby producing hydrogen sulphide ($H_2S$). $H_2S$ reacts with heavy metals such as lead or iron forming a black precipitate.
The experiment was carried out as follows:
1% Lead acetate solution was made in distilled water. Whatmann filter paper was taken and dipped in the lead acetate solution. The excess solution was drained and allowed to dry in laminar air Flow for 1 hour. Once dried, the paper was wrapped in aluminum foil and autoclaved for future use. Thereafter, 180 µL tryptic soy broth (TSB) was added with 0.1 % L-cysteine into the wells of 96-well plate. To this 20 uL of S. *hominis* culture ($10^6$ CFU/mL) was added. To this, treatments as shown in the table 5 below were added in concentrations indicated. Thereafter, lead acetate paper was placed on the wells and the plate was covered with a lid. The plate was incubated at 37°C overnight.

Visible blackening of lead acetate indicates the presence of $H_2S$. More blackening indicates more $H_2S$ is produced which correlates to presence of more bacteria. $H_2S$ readings were obtained for treatments outlined in table 5 and % reduction in $H_2S$ readings were calculated that correlates to proportionate reduction in S. *hominis.*

Table 4

| Example | Honokiol | Magnolol | Poly-DADMAC | $H_2S$ readings | % Reduction |
|---------|----------|----------|-------------|-----------------|-------------|
| I* | - | - | - | >10 | < 1 |
| J | 0.025 | 0.025 | - | 8 | < 5 |
| K | - | - | 0.005 | >10 | <1 |
| L | - | - | 0.01 | >10 | < 1 |
| M | | | 0.05 | 10 | < 1 |
| 6 | 0.025 | 0.025 | 0.005 | 2 | 80 |
| 7 | 0.025 | 0.025 | 0.01 | 2 | 80 |
| 8 | 0.05 | - | 0.05 | 2 | 80 |
| 9 | - | 0.05 | 0.05 | 2 | 80 |
| *only with TSB, no actives were added. | | | | | |

The data in table 5 above shows antimicrobial benefit is obtained when treatment with the compositions as per the present invention (examples 6 to 9) are carried out. All these examples show 80% reduction in $H_2S$ which means proportionate reduction in number of S. *hominis* thereby demonstrating antimicrobial benefit.

In conclusion, a composition comprising at least one biphenol wherein the biphenol is diallylbiphenol and at least one antimicrobial quaternary ammonium compound, provides antimicrobial benefit. Moreover, the antimicrobial benefit obtained is synergist and is obtained using minimal amounts of known antimicrobial actives.

## Claims

1. An antimicrobial composition comprising:

   a. at least one biphenol; and
   b. at least one antimicrobial quaternary ammonium compound
   wherein the biphenol is a diallylbiphenol,
   wherein the quaternary ammonium compound selected from benzethonium chloride, benzalkonium chloride, didecyl dimethyl ammonium chloride, cetylpyridinium chloride, cetyltrimethylammonium chloride, cetyltrimethylammonium bromide and polydiallyldimethylammonium chloride, wherein the amount of quaternary ammonium compound is in the range of 0.0001 to 5% by weight of the composition.

2. A composition as claimed in claim 1 wherein the biphenol is selected from 2,4'-biphenol, 2,2'-biphenol, 3,3'-biphenol, 4,4'-biphenol and mixtures thereof.

3. A composition as claimed in claim 2 wherein the diallylbiphenol is a (5-3'-diallyl)biphenol or a (5-5'-diallyl)biphenol.

4. A composition as claimed in claim 3 wherein the biphenol selected is honokiol.

5. A composition as claimed in any one of claims 1 to 4 further comprising from 1 to 80% by weight of a surfactant.

6. A composition as claimed in any one of claims 1 to 5 wherein the composition is in the form of a wash-off composition.

7. A composition as claimed in any one of claims 1 to 5 wherein the composition is in the form of a leave-on composition.

8. A non-therapeutic method of cleaning or disinfecting a surface comprising the step of applying a composition as claimed in any one of claims 1 to 7 on to a surface.

9. A non-therapeutic method as claimed in claim 8 wherein the composition is in the form of a wash-off composition and wherein there is an additional step of at least partially removing the applied composition.

10. A non-therapeutic method as claimed in claim 9 wherein the step of at least partially removing the composition is carried out in less than 5 minutes after the step of applying the composition on to the substrate.

11. Non-therapeutic use of a composition as claimed in any one claims 1 to 7 for antimicrobial benefit.

12. Use of a composition as claimed in any one of claims 1 to 7 for improved hand hygiene.


**Patentansprüche**

1. Antimikrobielle Zusammensetzung, umfassend:

   a. mindestens ein Biphenol; und
   b. mindestens eine antimikrobielle quaternäre Ammoniumverbindung,
   wobei das Biphenol ein Diallylbiphenol ist,
   wobei die quaternäre Ammoniumverbindung ausgewählt ist aus Benzethoniumchlorid, Benzalkoniumchlorid, Didecyldimethylammoniumchlorid, Cetylpyridiniumchlorid, Cetyltrimethylammoniumchlorid, Cetyltrimethylammoniumbromid und Polydiallyldimethylammoniumchlorid, wobei die Menge der quaternären Ammoniumverbindung in dem Bereich von 0,0001 bis 5%, bezogen auf das Gewicht der Zusammensetzung, liegt.

2. Zusammensetzung, wie im Anspruch 1 beansprucht, wobei das Biphenol aus 2,4'-Biphenol, 2,2'-Biphenol, 3,3'-Biphenol, 4,4'-Biphenol und Mischungen davon ausgewählt ist.

3. Zusammensetzung, wie im Anspruch 2 beansprucht, wobei das Diallylbiphenol ein (5-3'-Diallyl)biphenol oder ein (5-5'-Diallyl)biphenol ist.

4. Zusammensetzung, wie im Anspruch 3 beansprucht, wobei das ausgewählte Biphenol Honokiol ist.

5. Zusammensetzung, wie in irgendeinem der Ansprüche 1 bis 4 beansprucht, ferner umfassend 1 bis 80 Gewichts-% eines Tensids.

6. Zusammensetzung, wie in irgendeinem der Ansprüche 1 bis 5 beansprucht, wobei die Zusammensetzung in Form einer abwaschbaren Zusammensetzung vorliegt.

7. Zusammensetzung, wie in irgendeinem der Ansprüche 1 bis 5 beansprucht, wobei die Zusammensetzung in Form einer Leave-on-Zusammensetzung vorliegt.

8. Nicht-therapeutisches Verfahren zum Reinigen oder Desinfizieren einer Oberfläche, umfassend den Schritt des Auftragens einer Zusammensetzung, wie in irgendeinem der Ansprüche 1 bis 7 beansprucht, auf eine Oberfläche.

9. Nicht-therapeutisches Verfahren, wie im Anspruch 8 beansprucht, wobei die Zusammensetzung in Form einer abwaschbaren Zusammensetzung vorliegt und wobei dabei ein zusätzlicher Schritt des mindestens partiellen Entfernens der aufgetragenen Zusammensetzung erfolgt.

10. Nicht-therapeutisches Verfahren, wie im Anspruch 9 beansprucht, wobei der Schritt des mindestens partiellen Entfernens der Zusammensetzung in weniger als 5 Minuten nach dem Schritt des Auftragens der Zusammensetzung auf die Oberfläche durchgeführt wird.

11. Nicht-therapeutische Verwendung einer Zusammensetzung, wie in irgendeinem der Ansprüche 1 bis 7 beansprucht, zum antimikrobiellen Nutzen.

12. Zusammensetzung, wie in irgendeinem der Ansprüche 1 bis 7 beansprucht, zur Verwendung in einem Verfahren zur Verbesserung der Handhygiene.

**Revendications**

1. Composition antimicrobienne comprenant :

   a. au moins un bisphénol ; et
   b. au moins un composé d'ammonium quaternaire antimicrobien, dans laquelle le biphénol est un diallylbiphénol, dans laquelle le composé d'ammonium quaternaire est choisi parmi le chlorure de benzéthonium, le chlorure de benzalkonium, le chlorure de didécyldiméthylammonium, le chlorure de cétylpyridinium, le chlorure de cétyltriméthylammonium, le bromure de cétyltriméthylammonium et le poly(chlorure de diallyldiméthylammonium), dans laquelle la quantité du composé d'ammonium quaternaire est située dans la plage allant de 0,0001 à 5 % en poids de la composition.

2. Composition selon la revendication 1, dans laquelle le biphénol est choisi parmi le 2,4'-biphénol, le 2,2'-biphénol, le 3,3'-biphénol, le 4,4'-biphénol et leurs mélanges.

3. Composition selon la revendication 2, dans laquelle le diallylbiphénol est un (5,3'-diallyl)biphénol ou un (5,5'-diallyl) biphénol.

4. Composition selon la revendication 3, dans laquelle le biphénol choisi est l'honokiol.

5. Composition selon l'une quelconque des revendications 1 à 4, comprenant en outre de 1 à 80 % en poids d'un tensioactif.

6. Composition selon l'une quelconque des revendications 1 à 5, laquelle composition est sous la forme d'une composition à rincer.

7. Composition selon l'une quelconque des revendications 1 à 5, laquelle composition est sous la forme d'une composition sans rinçage.

8. Méthode non thérapeutique de nettoyage ou de désinfection d'une surface, comprenant l'étape d'application sur une surface d'une composition selon l'une quelconque des revendications 1 à 7.

9. Méthode non thérapeutique selon la revendication 8, dans laquelle la composition est sous la forme d'une composition à rincer, et dans laquelle il y a une étape supplémentaire dans laquelle la composition appliquée est éliminée au moins partiellement.

10. Méthode non thérapeutique selon la revendication 9, dans laquelle l'étape dans laquelle la composition est éliminée au moins partiellement est réalisée moins de 5 minutes après l'étape d'application de la composition sur le substrat.

11. Utilisation non thérapeutique d'une composition selon l'une quelconque des revendications 1 à 7 pour l'obtention d'un bénéfice antimicrobien.

12. Utilisation d'une composition selon l'une quelconque des revendications 1 à 7 pour améliorer l'hygiène des mains.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 11038797 A, Beiersdorf AG
- US 2009156551 A, DSM
- WO 0182922 A, Procter and Gamble
- US 2014328772 A, Colgate - Palmolive Co
- WO 2017194256 A1, Unilever

**Non-patent literature cited in the description**

- **CLARKE, P. H**. *J. gen. Microbiol.*, 1953, vol. 8, 397-407